# EUROPEAN PATENT APPLICATION

(11) **EP 3 357 355 A1**
(43) Date of publication of application: **08.08.2018**
(21) Application number: 18155407.2
(22) Date of filing: 06.02.2018
(51) Int. Cl.: A24F 47/00

(54) **ELECTRONIC CIGARETTE**

(30) Priority: 07.02.2017 CN 201720115508 U
(71) Applicant: Shenzhen IVPS Technology Co., Ltd., 518000 Shenzhen Guangdong (CN)
(72) Inventor: CHEN, Wen, Shenzhen, Guangdong 518000 (CN)
(74) Representative: IP-PAL Patent & Trademark Attorneys GmbH

(57) **Abstract**

The present disclosure discloses an electronic cigarette. The electronic cigarette comprises a first cover body 1 and a second cover body 2 interconnected with the first cover body. The first cover body comprises a main body 11 and a cover plate 10 rotatably connected to the main body. The main body is formed with an oil refilling passage 111 communicated with the second cover body. The cover plate covers or uncovers a port of the oil refilling passage. The present disclosure provides an electronic cigarette with convenient oil refilling.

## Description

### TECHNICAL FIELD

The present disclosure relates to an electronic cigarette.

### BACKGROUND

At present , oil refilling for an electronic cigarette is quiet complex. After screwing off an atomizer, oil spilling questions need to be noted during the total process of oil refilling. And it is quiet inconvenient.

### SUMMARY

The aim of the present disclosure is to provide an electronic cigarette so as to provide an electronic cigarette which could be refilled with oil easily.

To realize the above aim, the present disclosure provides an electronic cigarette. The electronic cigarette comprises a first cover body and a second cover body interconnected with the first cover body. The first cover body comprises a main body and a cover plate rotatably connected to the main body. The main body is formed with an oil refilling passage communicated with the second cover body, and the cover plate covers or uncovers a port of the oil refilling passage.

Preferably, the main body is formed with a first groove, the first groove is set close to the oil refilling passage. The cover plate is formed with a rotating axis, and the rotating axis is accommodated in the first groove.

Preferably, the main body is formed with a second groove, and the first groove is defined in a bottom wall of the second groove. The cover plate is buckling fit with the second groove.

Preferably, an end of the cover plate is formed with a protruding part, and a side wall of the second groove is formed with a fixing grooves. The protruding part is buckling fit with the fixing groove.

Preferably, the electronic cigarette further comprises a fixing ring, and the fixing ring is set in the first groove. The rotating axis is rotatably connected with the fixing ring.

Preferably, the electronic cigarette further comprises a first seal ring, and the first seal ring defines a through-hole. The bottom wall of the second groove is further formed with a third groove, and the first seal ring is set in the third groove. The oil refilling passage runs through a bottom wall of the third groove and the through-hole.

Preferably, the second cover body is formed with an accommodating groove, and the accommodating groove communicates with the oil refilling passage.

Preferably, the electronic cigarette further comprises a dripping nozzle, and one end of the dripping nozzle is connected with the first cover body. A second seal ring is set between the dripping nozzle and the first cover body.

Preferably, a core assembly is set inside the second cover body, and the core assembly communicates with the first cover body.

Preferably, the main body and the cover plate both are integrally formed.

In the technical solution of the present disclosure, the first cover body comprises the main body and the cover plate rotatably connected to main body. The main body is formed with the oil refilling passage communicated with the second cover body, and the cover plate covers or uncovers a port of the oil refilling passage. Users could refill oils through the oil refilling passage of the first cover body so as to refill oils for the electronic cigarette efficiently and conveniently.

### BRIEF DESCRIPTION OF THE DRAWINGS

For a better understanding of the embodiments of the present disclosure or the technical scheme in the prior art, accompanying drawings needed in the description of the embodiments or the prior art are simply illustrated below. Obviously, the accompanying drawings described below are some embodiments of the present disclosure. For the ordinary skill in the field, other accompanying drawings may be obtained according to the structure shown in these accompanying drawings without creative work.
Fig. 1 is a structural schematic view of the electronic cigarette of the present disclosure;
Fig. 2 is an exploded view of the first cover body in Fig. 1;
Fig. 3 is a schematic view of the first cover body in Fig. 1 under covering status;
Fig. 4 is a schematic view of the first cover body in Fig. 1 under uncovering status.

### Description of the reference number :

| Reference | Part | Reference | Part |
|---|---|---|---|
| 1 | first cover body | 114 | third groove |
| 10 | cover plate | 115 | fixing groove |
| 101 | rotating axis | 2 | second cover body |
| 102 | protruding part | 21 | accommodating groove |
| 11 | main body | 22 | core assembly |
| 111 | oil refilling passage | 3 | first seal ring |
| 112 | first groove | 4 | fixing ring |
| 113 | second groove | 5 | dripping nozzle |

The implementation of aims, the function features and the advantages of the present disclosure are described below in further detail in conjunction with embodiments with reference to the drawings.

### DETAILED DESCRIPTION OF PREFERRED EMBODIMENTS

A clear and complete description as below is provided for the technical scheme in the embodiments of the present disclosure in conjunction with the drawings in the embodiments of the present disclosure. Obviously, the embodiments described hereinafter are simply part embodiments of the present disclosure, but all the embodiments. All other embodiments obtained by the ordinary skill in the art based on the embodiments in the present disclosure without creative work are intended to be included in the scope of protection of the present disclosure..

It should be noted that all directional indications (such as top, bottom, left, right, front, behind...) in the embodiments of the present disclosure are merely to illustrate a relative position relation, a relative motion condition, etc. between each part in a certain state (for example, the state shown in the drawings). If the state changes, the directional indication changes accordingly.

In addition, if terms "first", "second", etc. appear in the present disclosure, they are merely for the purpose of description, but cannot be understood as the indication or implication of relative importance or as the implicit indication of the number of the designated technical features; therefore, features defined by "first" and "second" may specifically or implicitly include at least one such feature. In addition, technical schemes of each embodiment of the present disclosure may be combined mutually; however, this must be carried out on the basis that the ordinary skill in this field can implement the combination. When the combination of technical schemes has a conflict or cannot be implemented, it should be considered that such combination of technical schemes does not exist and is not in the scope of protection claimed by the present disclosure.

The present disclosure provides an electronic cigarette.

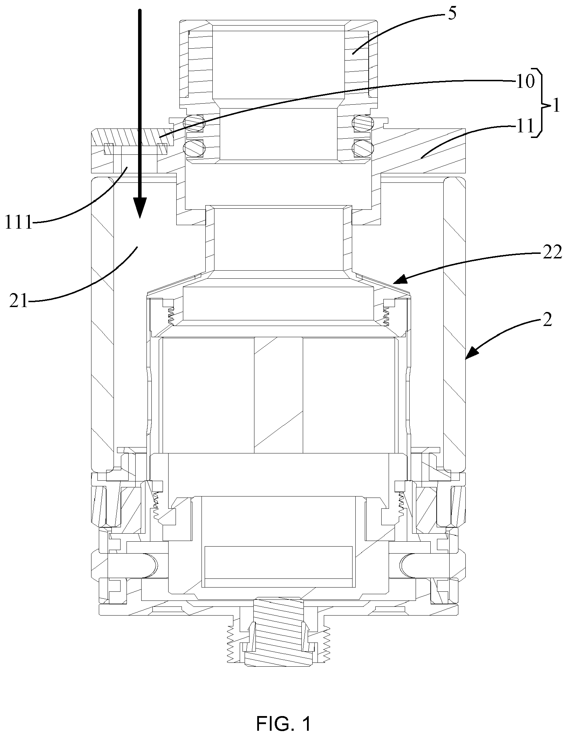

Referring to Fig 1 to Fig 4, in one embodiment of the present disclosure, the electronic cigarette comprises a first cover body 1 and a second cover body 2 interconnected with the first cover body 1. The first cover body 1 comprises a main body 11 and a cover plate 10 rotatably connected to the main body 11. The main body 11 is formed with an oil refilling passage 111 communicated with the second cover body 2. The cover plate 10 covers or uncovers a port of the oil refilling passage 111.

The main body 11 and the cover plate 10 are both made of plastic materials or light alloy materials and could be formed integrally.

In the technical solution of the present disclosure, the first cover body 1 comprises the main body 11 and the cover plate10 rotatably connected to the main body 11. The main body 11 is formed with an oil refilling passage111 communicated with the second cover body 2. The cover plate 10 covers or uncovers a port of the oil refilling passage 111. Users could refill oil through the oil refilling passage111 of the first cover body 1 in order to refill oil for electronic cigarette efficiently and conveniently.

Specially, the main body 11 in the present embodiment is formed with a first groove 112. The first groove 112 is set close to the oil refilling passage 111. The cover plate10 is formed with a rotating axis 101. The rotating axis 101 is accommodated in the first groove 112.

In the present embodiment, the cover plate 10 and the first groove 112 are both set on the top surface of the main body 11. The rotating axis 101 is rotatably connected to the first groove112 such that it could be conveniently uncovered and covered when users uncover or cover through rotating the cover plate 10.

In the present embodiment, the main body 11 is formed with a second groove 113. The first groove 112 is defined in a bottom wall of the second groove 113. The cover plate10 is buckling fit with the second groove 113 so as to efficiently fix the cover plate 10 when users rotate the cover plate 10.

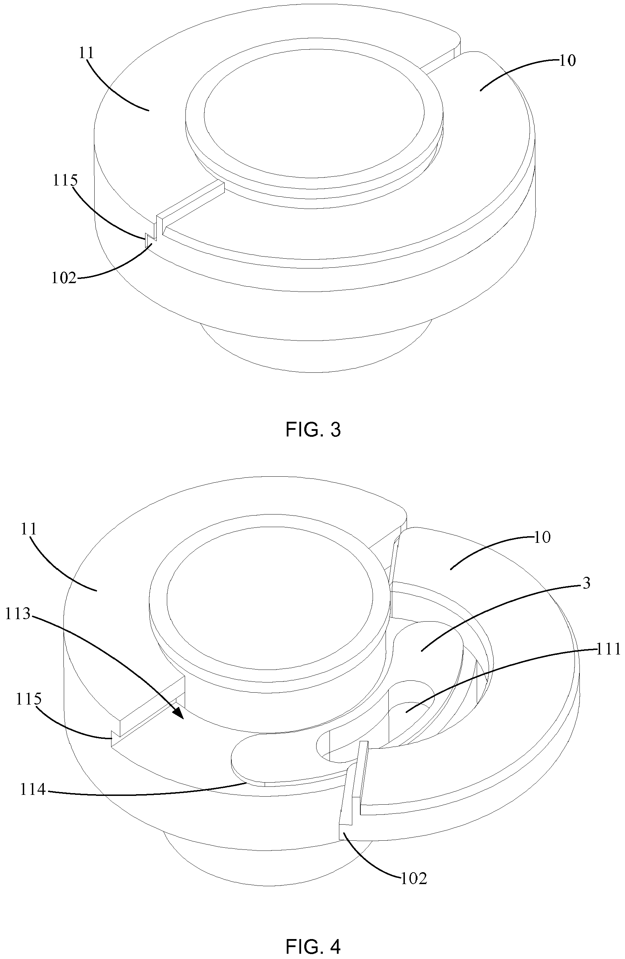

Furthermore, referring to Fig. 3, an end of the cover plate 10 in the present embodiment is formed with a protruding part 102. A side wall of the second groove 113 is formed with a fixing groove 115. The protruding part 102 is buckling fit with the fixing groove 115.

The protruding part 102 and the cover plate 10 are integrally formed during an molding process of the cover plate 10. In the present embodiment, the rotating axis 101 is located at one end of the cover plate 10, and the protruding part 102 is located at the other end of the cover plate 10. The fixing groove 115 is suitably connected with the protruding part 102. When the cover plate10 is under covering status , the protruding part102 is embedded in the fixing groove 115 in order to further fix the cover plate 10 efficiently.

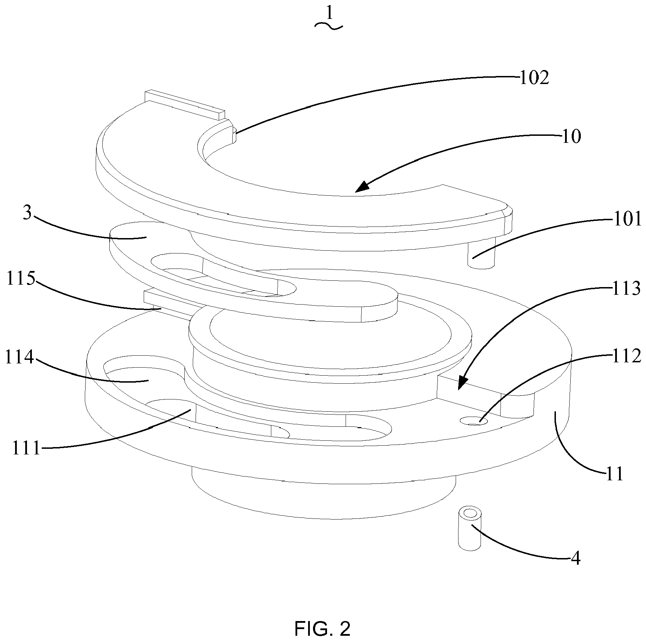

Referring to Fig. 2, in order to ensure conveniently rotating in the operation process, the electronic cigarette in the present embodiment further comprises a fixing ring 4. The fixing ring 4 is set in the first groove 112. The rotating axis 101 is rotatably connected with the fixing ring 4.

The fixing ring 4 is integrally molding formed. The fixing ring 4 is accommodated in the first groove 112. The axial center of the fixing ring 4 defines a through-hole, and the rotating axis 101 is embedded in the through-hole. In the present embodiment, fixing ring 4 is sleeve-connected on the rotating axis 101 such that it is steadier when users rotate the cover plate 10 and the cover plate 10 is replaceable when damaged.

Preferably, referring to Fig.3 to Fig.4, the electronic cigarette of the present embodiment further comprises a first seal ring 3. The first seal ring 3 defines a through-hole. The bottom wall of the second groove 113 is further formed with a third groove 114. The first seal ring 3 is set in the third groove 114. The oil refilling passage 111 runs through the bottom wall of the third groove 114 and the through-hole.

The first seal ring 3 is integrally molding formed an integration structure of injection molding. The third groove 114 and the main body 11 are integrally formed during the molding process of the main body 11. In the present embodiment, the first seal ring 3 is embedded in the third groove 114. After the cover plate 10 is uncovered, users can refill oils through the through-hole of the first seal ring 3. After the cover plate 10 is covered, the first seal ring 3 is between the cover plate 10 and the main body 11 in order to realize efficient sealing and shockproof.

Referring to Fig. 1, the second cover body 2 of the present embodiment is formed with an accommodating groove 21. The accommodating groove 21 communicates with the oil refilling passage 111.

The second cover body 2 is formed with an oil tank. An accommodating groove 21 is formed inside the oil tank. In the present embodiment, one end of the oil refilling passage 111 is connected with the oil tank, and the other end is an oil inlet.

The electronic cigarette of the present embodiment further comprises a dripping nozzle 5. An end of the dripping nozzle 5 is connected with the first cover body 1. A second seal ring is set between the dripping nozzle 5 and the first cover body 1.

The dripping nozzle 5 communicates with the first cover body 1. An end of the dripping nozzle 5 is rotatably connected to the first cover body 1 by a way of screwing connection. Specially, the way of screwing connection is that the first cover body 1 is set with an threaded hole, and an end of the dripping nozzle 5 is set with an external screw thread. The dripping nozzle 5 and the first cover body 1 are in threaded connection. In the present embodiment, the second seal ring is set on the connection part between the dripping nozzle 5 and the first cover body 1 in order to realize tightly connection efficiently.

Furthermore, a core assembly 22 is set inside the second cover body 2 of the present embodiment. The core assembly 22 communicates with the first cover body 1.

The core assembly 22 is set close to the oil tank. The core assembly 22 is a vapor device. Smoke is produced by core assembly 22, and passes through the first cover body 1, thus discharged from the dripping nozzle 5.

The above are preferred embodiments of the present disclosure merely and are not intended to limit the patent scope of the present disclosure. Any equivalent structures made according to the description and the accompanying drawings of the present disclosure without departing from the idea of the present disclosure, or any equivalent structures applied in other relevant technical fields directly or indirectly are intended to be included in the patent protection scope of the present disclosure.

## Claims

1. An electronic cigarette, wherein the electronic cigarette comprises a first cover body and a second cover body interconnected with the first cover body, the first cover body comprises a main body and a cover plate rotatably connected to the main body, the main body is formed with an oil refilling passage communicated with the second cover body, the cover plate covers or uncovers a port of the oil refilling passage.

2. The electronic cigarette according to claim 1, wherein the main body is formed with a first groove, the first groove is set close to the oil refilling passage; the cover plate is formed with a rotating axis, the rotating axis is accommodated in the first groove.

3. The electronic cigarette according to claim 2, wherein the main body is formed with a second groove, the first groove is defined in a bottom wall of the second groove, the cover plate is buckling fit with the second groove.

4. The electronic cigarette according to claim 3, wherein an end of the cover plate is formed with a protruding part, a side wall of the second groove is formed with a fixing grooves, the protruding part is buckling fit with the fixing groove.

5. The electronic cigarette according to claim 2, wherein the electronic cigarette further comprises a fixing ring, the fixing ring is set in the first groove, the rotating axis is rotatably connected with the fixing ring.

6. The electronic cigarette according to claim 3, wherein the electronic cigarette further comprises a first seal ring, the first seal ring defines a through-hole, the bottom wall of the second groove is further formed with a third groove, the first seal ring is set in the third groove, the oil refilling passage runs through a bottom wall of the third groove and the through-hole.

7. The electronic cigarette according to claim 1, wherein the second cover body is formed with an accommodating groove, and the accommodating groove communicates with the oil refilling passage.

8. The electronic cigarette according to any of claims 1 to 7, wherein the electronic cigarette further comprises a dripping nozzle, one end of the dripping nozzle is connected with the first cover body, a second seal ring is set between the dripping nozzle and the first cover body.

9. The electronic cigarette according to claim 7, wherein a core assembly is set inside the second cover body and the core assembly communicates with the first cover body.

10. The electronic cigarette according to claim 1, wherein the main body and the cover plate are integrally formed.
